# EUROPEAN PATENT APPLICATION

(11) **EP 3 128 320 A1**
(43) Date of publication of application: **08.02.2017**
(21) Application number: 15772882.5
(22) Date of filing: 13.03.2015
(51) Int. Cl.: G01N 27/447

(54) **SEPARATION MEDIUM CASSETTE FOR SAMPLE SEPARATION ADSORPTION AND ANALYSIS DEVICE FOR SAMPLE SEPARATION ADSORPTION**

(30) Priority: 31.03.2014 JP 2014074022
(71) Applicant: Sharp Kabushiki Kaisha, Sakai City, Osaka 590-8522 (JP)
(72) Inventor: TOMINAGA, Taiga, Sakai City, Osaka 590-8522 (JP); UNUMA, Yutaka, Sakai City, Osaka 590-8522 (JP); OHKI, Hiroshi, Sakai City, Osaka 590-8522 (JP); GOTO, Shinichi, Sakai City, Osaka 590-8522 (JP)
(74) Representative: Treeby, Philip David William
(86) International application number: PCT/JP2015/057557
(87) International publication number: WO 2015/151768

(57) **Abstract**

Provided is a feature for performing more accurate analysis in a sample separation adsorption method. A gel cassette (22) is provided with a separation medium (19) for separating out a sample by electrophoresis and discharging the sample, a first insulating portion (13a) and a second insulating portion (13b) sandwiching the separation medium, and warping prevention portions (20) that are inserted between the first insulating portion (13a) and the second insulating portion (13b), and that mitigate warping of the first insulating portion (13a) and the second insulating portion (13b).

## Description

### TECHNICAL FIELD

The present invention relates to a separation medium cassette for sample separation adsorption, and an analysis device for sample separation adsorption including the separation medium cassette for sample separation adsorption.

### BACKGROUND ART

In recent years, direct blotting has been developed, which discharges samples separated in a separation medium by way of electrophoresis from an end face of the separation medium and transfers directly to a transfer membrane opposing the end face of the separation medium.

Patent Document 1 discloses a cassette that electrophoretically separates molecular fragments in solution. The cassette described in Patent Document 1 includes (a) a gelatinous substrate containing molecular fragments for separation at a first end thereof and configured sufficient to concentrate similar separated molecular fragments (sample) at a second end thereof; and (b) a containment means which supports the gelatinous substrate. The components separated in the gel cassette transfer to a moving membrane at a discharge port (slot 40 in Patent Document 1).

Patent Document 1: Japanese Published Translation of PCT International Publication for Patent Applications "Japanese Unexamined Patent Application (Translation of PCT Publication), Publication No. H9-501774 (published February 18, 1997)"

### DISCLOSURE OF THE INVENTION

### Problems to be Solved by the Invention

However, with the gel cassette according to Patent Document 1, since the moving membrane contacts the gel cassette, the cassette may warp. Since warping of the gel cassette greatly influences the test results, it is not possible to obtain accurate analysis results if the cassette warps. In other words, there are problems in that the gap between gel plates is completely eliminated and transfer is inhibited, and the width of the gap between both ends of the gel plate and the central part varies and the transfer rate is no longer constant in the width direction.

The present invention has been made taking account of the above-mentioned issues, and has a main object of providing technology for performing more accurate analysis in a sample separation adsorption method.

### Means for Solving the Problems

In order to solve the above-mentioned problem, a separation medium cassette for sample separation adsorption according to a first aspect of the present invention includes: a separation medium that separates sample by way of electrophoresis and discharges from an end face thereof; a first insulating portion and a second insulating portion that form a pair sandwiching the separation medium; and a warping prevention portion that is inserted between the first insulating portion and the second insulating portion, and suppresses the first insulating portion and the second insulating portion from warping so as to approach each other.

### Effects of the Invention

According to the present invention, it is possible to perform more accurate analysis in a sample separation adsorption method.

### BRIEF DESCRIPTION OF THE DRAWINGS

FIG. 1 is a cross-sectional view showing an outline structure of an analysis device to which a gel cassette according to an embodiment (first embodiment) of the present invention is inserted;
FIG. 2 is a perspective view showing an outline structure of a gel cassette and transfer membrane according to an embodiment (first embodiment) of the present invention;
FIG. 3 provides views showing the shape of a gel cassette according to an embodiment (first embodiment) of the present invention, with (a) being a front view of the gel cassette, and (b) being a plan view showing an end face at which the gel cassette discharges sample;
FIG. 4 provides views showing the shape of a gel cassette according to an embodiment (second embodiment) of the present invention, with (a) being a perspective view showing an outline structure of the gel cassette and transfer membrane, and (b) being a front view of the gel cassette;
FIG. 5 provides views showing the shape of a gel cassette according to an embodiment (third embodiment) of the present invention, with (a) being a perspective view showing an outline structure of the gel cassette and transfer membrane, and (b) being a front view of the gel cassette; and
FIG. 6 provides views showing the shape of a gel cassette according to an embodiment (fourth embodiment) of the present invention, with (a) being a perspective view showing an outline structure of the gel cassette and transfer membrane, and (b) being a front view of the gel cassette.

### PREFERRED MODE FOR CARRYING OUT THE INVENTION

A separation medium cassette for sample separation adsorption according to the present invention includes: a separation medium that separates sample by way of electrophoresis and discharges from an end face thereof; a first insulating portion and a second insulating portion that form a pair sandwiching the separation medium; and a warping prevention portion that is inserted between the first insulating portion and the second insulating portion, and suppresses the first insulating portion and the second insulating portion from warping so as to approach each other.

In the present disclosure, sample separation adsorption method indicates technology for transferring a separated sample to a transfer membrane by arranging the transfer membrane so as to oppose an end face of a separation medium separating a sample by way of electrophoresis, positioned downstream in the running direction of electrophoresis, and then causing the sample separated by way of electrophoresis to discharge from this end face as is. In order to continuously perform transfer to the transfer membrane, the transfer membrane is relatively moved in relation to the end face. According to the sample separation adsorption method, it is possible to perform the separation of sample and transfer to the transfer membrane in few steps.

In the present disclosure, separation medium cassette for sample separation adsorption indicates a structure including a separation medium and an insulating portion that retains the separation medium, and is inserted in an analysis device for conducting the sample separation adsorption method.

In the present disclosure, sample is a substance to be analyzed by electrophoresis and transfer. As the sample, a preparation from biological material (e.g., biont, body fluid, cell strain, tissue culture, or tissue fragment) can be suitably used, and in particular, protein samples, DNA samples and RNA samples can be suitable used. These samples may be stained (immunostaining or fluorescent labeling) by a fluorescent sample or the like in advance. In addition, the sample is preferably subjected to pretreatment such as purification and SDS processing.

In the present disclosure, separation medium indicates a medium that can separate the sample by way of electrophoresis, and is not limited to these; however, for example, it is possible to exemplify gels such as polyacrylamide gel and agarose gel. In addition, it may be of a structure in which ultrafine posts (nano-pillars) are provided standing.

In the present disclosure, insulating portion is an insulating body, and retains the separation medium by sandwiching. As the insulating body, although glass having a hydrophilic surface can be suitably used, for example, it is not limited thereto, and it is possible to use an insulating material such as acrylic resin, polycarbonate resin, polystyrene resin, PET resin, vinyl chloride resin, and ceramics.

In the present disclosure, warping prevention portion indicates a structure inserted between a first insulating portion and second insulating portion, and suppressing the first insulating portion and second insulating portion from warping so as to approach each other.

As the substance constituting the warping prevention portion, for example, it is possible to use plastic resins such as polymethylmethacrylate (acrylic), polystyrene, polyethylene, polypropylene, polyethylene terephthalate (PET) and polyether ether ketone, glass or silicon; however, it is not limited to these, and it is possible to use a substance that is an insulating body. It should be noted that, in the case of forming the separation medium in a state in which the warping prevention portion is inserted between the first insulating portion and second insulating portion, it is preferable for the warping prevention portion not to inhibit formation of the separation medium (e.g., polymerization of gel), and to be a substance of high gas barrier property.

In addition, although the form of the warping prevention portion is not particularly limited, for example, it can be established in forms such as a column shapes like a cylinder, elliptical column, triangular column, square column, and polygonal columns of five or more sides, and wall-like.

Furthermore, the warping prevention portion may be integrated by adhesion or the like to at least one of the first insulating portion and second insulating portion, or may be inserted between the first insulating portion and second insulating portion when forming the separation medium.

Moreover, the analysis device for sample separation adsorption according to the present invention includes the above-mentioned separation medium cassette for sample separation adsorption, and a moving portion that causes the transfer membrane to move so as to pass through a position opposing an end face in the direction of electrophoresis of the separation medium.

In the present disclosure, transfer membrane is a membrane that catches (blots or transfers) the separated sample while retaining the separation pattern, and is also simply called a film, transfer membrane, membrane or filter, due to a membrane of thin sheet form being used generally. As the transfer membrane, for example, although nitrocellulose membranes, nylon membranes, polyvinylidene fluoride (PVDF) membranes, etc. can be exemplified, it is not limited thereto, and so long as being a membrane that can catch the separation pattern of a sample from the separation medium by way of a capillary method, electroblotting method, etc., it can be suitably employed.

In the present disclosure, analysis device for sample separation adsorption is a device for analyzing sample by way of a sample separation adsorption method, and indicates a device that performs from a sample being introduced until the transfer thereof to the transfer membrane with one device.

### (First Embodiment)

Hereinafter, an embodiment of the present invention will be explained in detail.

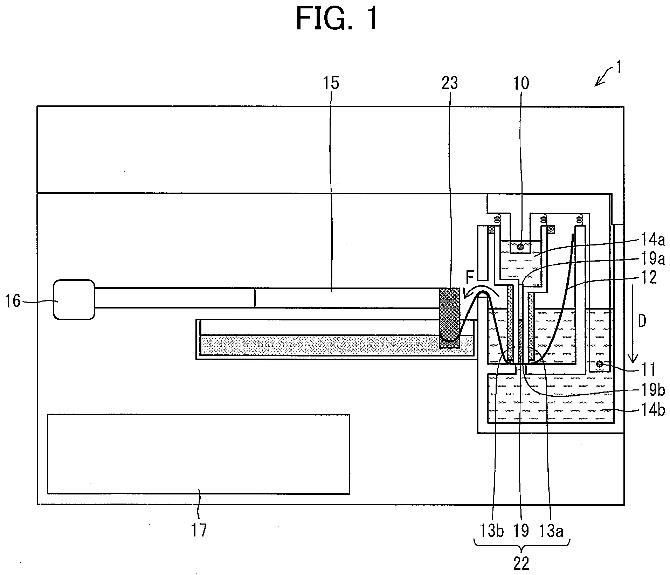

FIG. 1 is a cross-sectional view showing an outline structure of an analysis device (analysis device for sample separation adsorption) 1 to which a gel cassette (separation medium cassette for sample separation adsorption) 22 according to an embodiment (first embodiment) of the present invention is inserted. As shown in FIG. 1, the analysis device 1 includes a cathode 10, anode 11, transfer membrane 12, buffer solution tank 14a, buffer solution tank 14b, arm 15, motor 16, power unit 17, gel cassette (separation medium cassette for sample separation adsorption) 22, and connecting part 23.

The gel cassette 22 has a structure in which a first insulating portion 13a and second insulating portion 13b form a pair to sandwich a gel (separation medium) 19.

Inside of the analysis device 1, the gel cassette 22 is arranged in a vertical direction, and the buffer solution tank 14a is arranged on an upstream side (vertically upper side) in the running direction D of electrophoresis of the gel cassette 22. The gel cassette 22 and buffer solution tank 14a are arranged so that an end face 19a of the gel 19 of the gel cassette 22 contacts an opening part of the buffer solution tank 14a provided at a bottom part. The cathode 10 is arranged inside of the buffer solution tank 14a, and buffer solution is filled so as to immerse the cathode 10 and the end face 19a of the gel 19 in the buffer solution tank 14a.

The buffer solution tank 14b is arranged so as to surround the gel cassette 22 on a downstream side (vertically lower side) in the running direction D of electrophoresis of the gel cassette 22, and the end face 19b of the gel 19 is exposed within the buffer solution tank 14b. The anode 11 is arranged inside of the buffer solution tank 14b, and buffer solution is filled so as to immerse the anode 11 and the end face 19b of the gel in the buffer solution tank 14b.

As the buffer solution filled in the buffer solution tank 14a and the buffer solution tank 14b, it is possible to use any buffer solution having electrical conductivity; however, as the buffer solutions usable in electrophoresis, for example, it is possible to use buffer solutions such as a Tris/glycine-based buffer solution, acetic acid buffer solution, sodium carbonate-based buffer solution, CAPS buffer solution, Tris/boric acid/EDTA buffer solution, Tris/acetic acid/EDTA buffer solution, MOPS, phosphoric acid buffer solution, and Tris/tricine-based buffer solution.

The cathode 10 and anode 11 are formed from materials having electrical conductivity such as metals. As the material forming the cathode 10 and anode 11, platinum is preferable from the view point of suppressing ionization of the electrodes, for example.

The transfer membrane 12 has a sheet form, and comes to move through a position opposing the end face 19b positioned on a downstream side in the running direction D of electrophoresis of the gel 19. The connecting part 23 consisting of resin or the like is provided at an end in the running direction F of the transfer membrane 12, and is connected to the arm 15, which is driven by the motor 16. Therefore, by the motor 16 driving the arm 15, it is possible to cause the transfer membrane 12 to relatively move in relation to the end face 19b of the gel 19. For example, in the cross-sectional view shown in FIG. 1, the transfer membrane 12 moves towards the running direction F so as to pass through a position opposing the end face 19b of the gel 19, by the motor 16 pulling the arm 15 in a direction approaching the motor 16 (left direction in FIG. 1). It should be noted that the transfer membrane 12 may move (slide) while contacting the end face 19b. In addition, in the case of a porous film (not illustrated) covering the end face 19b being provided to the end face 19b of the separation medium 19, it may move while contacting with the porous film.

The motor 16 drives the arm 15 that is connected. The motor 16 is sufficient so long as being a motor that can drive the arm 15, so as to relatively move the transfer membrane 12 in relation to the end face of the gel cassette 22. As an example of a motor used as the motor 16, it is possible to exemplify a stepping motor having a 0.36° step angle, and 500 kHz maximum frequency.

The power unit 17 supplies electricity required in order for the motor 16, etc. to operate, as well as providing voltage to apply between
It should be noted that the voltage applied between the cathode 10 and anode 11 may be a constant voltage, may be a constant current, or may switch between these.

### (Operation of Analysis Device 1)

Operation of the analysis device 1 will be explained hereinafter.

The sample is introduced to the gel cassette 22, and electrophoresis starts by flowing current between the cathode 10 and anode 11. The sample introduced to the gel cassette 22 is separated by way of electrophoresis. In other words, each component contained in the sample undergoes phoresis at different speeds in the separation medium 19 according to the characteristics (e.g., molecular weight) of each component, and is separated into every component. Then, components having a fast phoretic velocity are discharged early from the end face 19b, and components having a slow phoretic velocity are discharged later from the end face 19b. The transfer membrane 12 is moved so as to pass through a position opposing the end face 19b by the motor 16 driving the arm 15. Each component of the sample discharged from the end face 19b is transferred consecutively at different sites on the transfer membrane 12 according to the order of discharging thereof. In the above way, it is possible to successfully transfer the separated sample to the transfer membrane. It should be noted that the transfer membrane to which the separated sample is transferred can be offered for further analysis such as immunostaining, for example.

At this time, a force causing to warp so as to approach the second insulating portion 13b may act on the first insulating portion 13a by the transfer membrane 12 moving. In particular, the first insulating portion 13a and second insulating portion 13b tend to easily warp even with slight force, since (i) it is necessary to form to be thin to some extent for heat dissipation, and (ii) in order to thin the bands of sample transferred to the transfer membrane 12, they may be formed in a tapering (tapered) shape towards the running direction D of electrophoresis. Herein, the gel cassette 22 according to the present embodiment includes a warping prevention part 20 that is inserted between the first insulating portion 13a and second insulating portion 13b, and suppresses warping such that the first insulating portion 13a and second insulating portion 13b approach each other, whereby it is possible to suppress the first insulating portion 13a and second insulating portion 13b from warping, and thus perform analysis more accurately. Hereinafter, the gel cassette 22 according to the present embodiment will be explained in detail.

### (Gel Cassette 22)

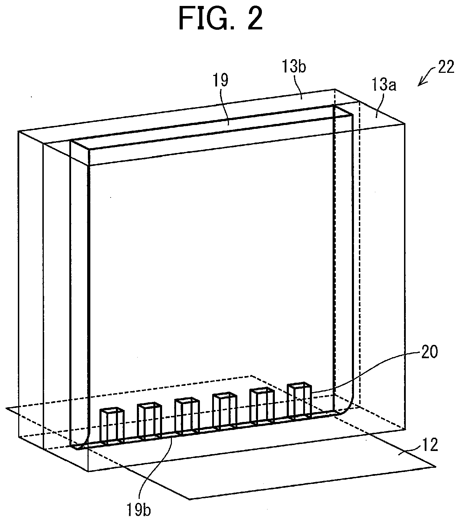

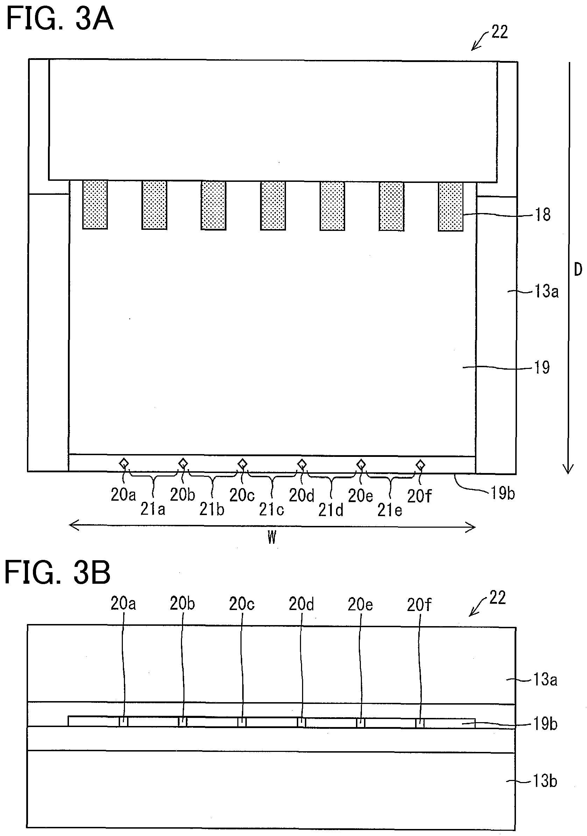

FIG. 2 is a perspective view showing an outline structure of the gel cassette 22 and transfer membrane according to an embodiment (first embodiment) of the present invention. FIG. 3 provides views showing the shape of the gel cassette 22 according to an embodiment (first embodiment) of the present invention, with (a) being a front view of the gel cassette 22, and (b) being a plan view showing the end face at which the gel cassette 22 discharges sample.

As shown in FIG. 2, the gel 19 is retained in the gel cassette 22 by being sandwiched by the first insulating portion 13a and second insulating portion 13b. The end face 19b in the electrophoresis direction D of the gel 19 opposes the transfer membrane 12. The warping prevention portion 20 is inserted in an end in the electrophoresis direction D (end face 19b side) between the first insulating portion 13a and second insulating portion 13b.

According to this configuration, even in a case of a force such that brings together is acting on the first insulating portion 13a and second insulating portion 13b, the warping prevention portion 20 inserted between the first insulating portion 13a and second insulating portion 13b works like a prop to suppress the first insulating portion 13a and second insulating portion 13b from warping. It is thereby possible to perform more accurate analysis. In addition, in order to be able to suppress the warping of the first insulating portion 13a and second insulating portion 13b by way of the warping prevention portion 20, it is possible to suitably use a material such as acrylic resin which has low cost and low rigidity compared to glass, etc., as the first insulating portion 13a and second insulating portion 13b.

It should be noted that the warping prevention portion 20 may be a shape connecting with both the first insulating portion 13a and second insulating portion 13b, in a state in which the first insulating portion 13a and second insulating portion 13b are not warping; however, it is may be a shape in which one among the first insulating portion 13a and second insulating portion 13b is not connected. In a state in which the first insulating portion 13a and second insulating portion 13b are not warping, in the case of the warping prevention portion 20 being a form that does not connect with one among the first insulating portion 13a and second insulating portion 13b, the first insulating portion 13a and second insulating portion 13b can warp so as to approach each other initially; however, by the first insulating portion 13a and second insulating portion 13b warping so as to approach each other, when the warping prevention portion 20 connects (abuts) with both of the first insulating portion 13a and second insulating portion 13b, the warping prevention portion 20 acts like a prop as mentioned above, and can suppress the first insulating portion 13a and second insulating portion 13b from warping any more than this.

In addition, as shown in FIG. 3(a), the gel cassette 22 includes a sample introduction port 18 for introduction of the sample to the gel 19. It should be noted that the configuration of the sample introduction port 18 is not limited thereto, and may be a configuration provided to the second insulating portion 13b.

The sample is introduced to the gel 19 from the sample introduction port 18. The method of introducing sample is not particularly limited; however, it can be introduced by determining the quantity with a Pipetman, for example. In addition, in the case of performing electrophoresis in a second dimension in two-dimensional electrophoresis, the sample may be introduced by plugging an IPG gel after performing isoelectric focusing into the sample introduction port 18.

The sample introduced to the sample introduction port 18 is separated by way of electrophoresis, and is discharged from the end face 19b of the gel 19 positioned downstream in the running direction D. In the present disclosure, the path on which a sample separated by electrophoresis flows is called a lane.

As shown in FIGS. 2, 3(a) and 3(b), the warping prevention portion 20 is configured by warping prevention parts 20a to 20f. The warping prevention parts 20a to 20f are inserted between the ends in the electrophoresis direction D of the first insulating portion 13a and second insulating portion 13b. According to this configuration, it is possible to more suitably suppress the end face 19b of the gel 19 from deforming. It is thereby possible to further reduce the negative influence on the separation of the sample in the gel 19 and the discharge of sample from the end face 19b of the gel 19, and thus perform more accurate analysis.

In addition, as shown in FIGS. 3(a) and 3(b), the gel 19 has a plurality of lanes (e.g., lanes 21a to 21f), and separates samples in every lane, and the warping prevention parts 20a to 20f are inserted between adjacent lanes. For example, the warping prevention portion 20b is inserted between the lane 21a and lane 21b, and the warping prevention portion 20c is inserted between the lane 21b and lane 21c. According to this configuration, the warping prevention parts 21a to 21f are inserted at positions not hindering the separation of sample flowing in the lanes 21a to 21f and discharge of separated sample. It is thereby possible to perform accurate analysis.

In addition, as shown in in FIGS. 2 and 3(a), and FIG. 3(b), at least a part of the warping prevention parts 20a to 20f (warping prevention parts 20c and 20d) is inserted at a central part in the width direction W, which is orthogonal to the electrophoresis direction D. It should be noted that, in the present disclosure, central part in the width direction W, which is orthogonal to the electrophoresis direction D, indicates the matter of the central vicinity in the width direction W that is orthogonal to the electrophoresis direction D, e.g., the portion in the middle arrived at by trisecting the width direction W is defined as the central part. Herein, the gel cassette 22 is fixed to the analysis device 1 at the outer circumference usually; therefore, the central part tends to warp easily. According to this configuration, by at least a part of the warping prevention parts 20a to 20f (warping prevention parts 20c and 20d) being inserted at the central part in the width direction D that is orthogonal to the electrophoresis direction D, it is possible to suitably suppress the first insulating portion 13a and second insulating portion 13b from warping by reinforcing the central parts of the first insulating portion 13a and second insulating portion 13b, which easily warp. It is thereby possible to perform more accurate analysis.

### (Regarding storage form of gel cassette)

It should be noted that the gel cassette 22 may assume a form for storage over a long time in a state not inserted into the analysis device 1.

For example, it may include a sample introduction port lid (not illustrated) that covers the sample introduction port 18. As the material of the sample introduction port lid, for example, although an acrylic resin for which processing is easy can be suitably used, it is not limited thereto, and it is possible to use insulating materials such as acrylic resin, polycarbonate resin, polystyrene resin, PET resin, vinyl chloride, and ceramic.

In addition, a package for wrapping the entirety of the gel cassette 22 may be included. Inside this package, a reinforcing material for supplementing the strength of the gel cassette 22, buffer solution for preventing drying of the gel 19, etc. may be placed.

### (Regarding Modified Example of Analysis Device)

The analysis device 1 may further include a guide mechanism (roller, etc.) for defining the movement path of the transfer membrane 12.

In addition, in the analysis device 1, although the gel cassette 22 is arranged in the vertical direction, the present embodiment is not limited thereto. The gel cassette 22 may be arranged in the horizontal direction, for example.

### (Second Embodiment)

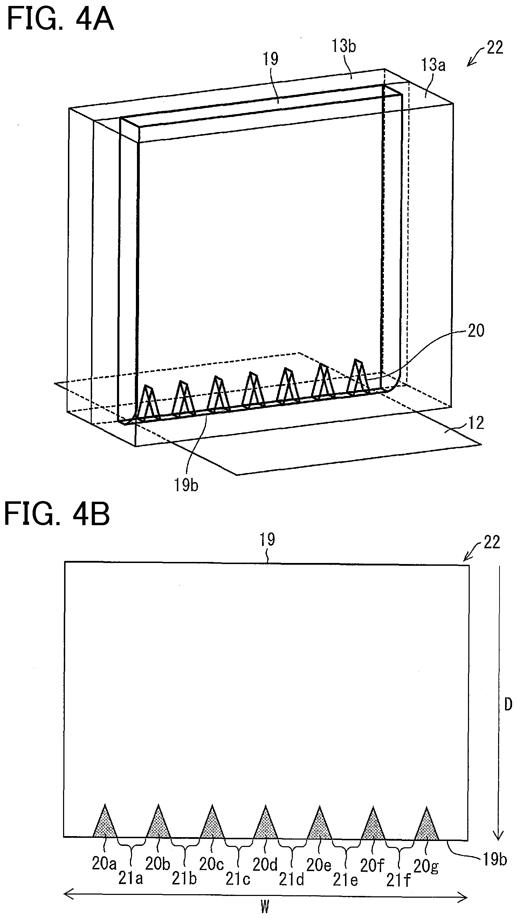

Another embodiment (second embodiment) of the present invention is as follows when explaining based on FIG. 4. It should be noted that, for convenience of explanation, the same reference symbols are assigned for members having the same function as members explained in the embodiment, and explanations thereof will be omitted. FIG. 4 provides views showing the shape of the gel cassette 22 according to an embodiment (second embodiment) of the present invention, with (a) being a perspective view showing an outline structure of the gel cassette 22 and transfer membrane 12, and (b) being a front view of the gel cassette 22.

As shown in FIGS. 4(a) and (b), the warping prevention portion 20 is configured by the warping prevention parts 20a to 20g. The warping prevention parts 20a to 20g have a shape thicker at one end along the electrophoresis direction D. In the present disclosure, shape thicker at one end along the electrophoresis direction D indicates a shape in which a length in the width direction W elongates as advancing in the electrophoresis direction D.

According to this configuration, in the lanes on both sides of the warping prevention parts 20a to 20g having the shape thicker at one end along the electrophoresis direction D (e.g., lanes 21a to 21f), the width of the lane narrows as advancing in the electrophoresis direction D (end face 19b side). For example, in the lanes 21a and 21b, which are lanes on both sides of the warping prevention part 20b, the width of the lane narrows as advancing to the side of the end face 19b. Therefore, the sample is led into a narrower space as advancing in the lane, to be concentrated. The distance between this concentrated sample and samples flowing in adjacent lanes (e.g., lanes 21a and 21c for lane 21b) is thereby ensured. In the above way, it is possible to concentrate the sample and prevent the mixing of samples between lanes, and thus perform more detailed analysis. In other words, as the phoresis distance of sample lengthens, the sample scatters and the band of the sample transferred to the transfer membrane 12 widens in the width direction W, and in the case of the bands of samples flowing in adjacent lanes overlapping, the quantitative analysis of results may become difficult; however, according to the present embodiment, it is possible to successfully segregate between bands by narrowing the width of bands of samples.

In addition, with the gel cassette 22 according to the present embodiment, similarly to the gel cassette 22 of the first embodiment, the warping prevention portion 20 is inserted between the ends in the electrophoresis direction D of the first insulating portion 13a and second insulating portion 13b. According to this configuration, since it is possible to suitably suppress deformation of the end face 19b of the gel 19, it is possible to further reduce the negative influence on discharging of sample from the end face 19b, and thus perform more accurate analysis.

In addition, with the gel cassette 22 according to the present embodiment, similarly to the gel cassette 22 of the first embodiment, at least a part of the warping prevention parts 20a to 20g (warping prevention parts 20c, 20d and 20e) is inserted at a central part in the width direction W that is orthogonal to the electrophoresis direction D. According to this configuration, it is possible to suitably suppress the first insulating portion 13a and second insulating portion 13b from warping by reinforcing the central parts of the first insulating portion 13a and second insulating portion 13b, which easily warp. It is thereby possible to perform more accurate analysis.

### (Third Embodiment)

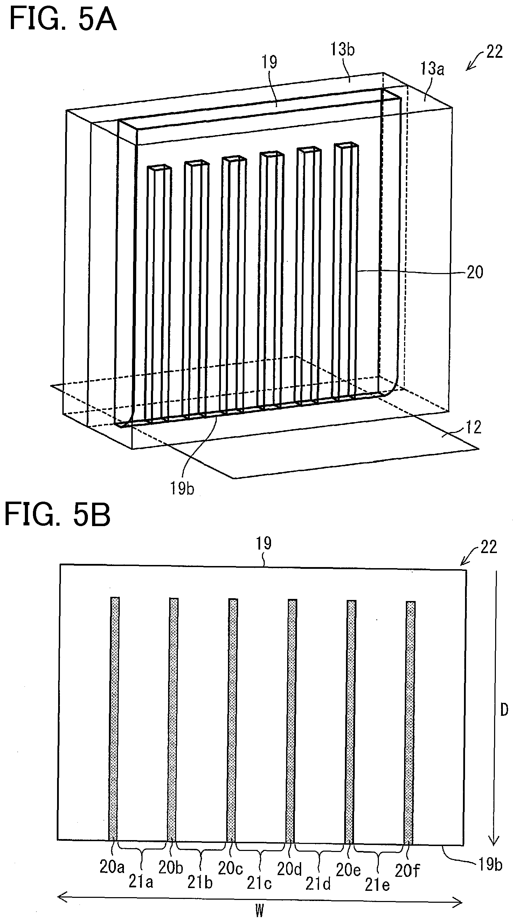

Another embodiment (third embodiment) of the present invention is as follows when explaining based on FIG. 5. It should be noted that, for convenience of explanation, the same reference symbols are assigned for members having the same function as members explained in the embodiment, and explanations thereof will be omitted. FIG. 5 provides views showing the shape of the gel cassette 22 according to an embodiment (third embodiment) of the present invention, with (a) being a perspective view showing an outline structure of the gel cassette 22 and transfer membrane 12, and (b) being a front view of the gel cassette 22.

As shown in FIGS. 5(a) and (b), the warping prevention portion 20 is configured by the warping prevention parts 20a to 20f. The warping prevention parts 20a to 20f are provided so as to divide between adjacent lanes. For example, the warping prevention part 20b is provided so as to divide between the adjacent lanes 21a and 21b. In the present disclosure, divide between adjacent lanes indicates the matter of the length in the running direction D of electrophoresis being at least a predetermined length, and as an example of a predetermined length, a case of defining as half the length of the gel 19 in the running direction D of electrophoresis can be exemplified.

According to this configuration, the sample is separated and discharged without crossing between lanes. It is thereby possible to perform more detailed analysis by preventing the mixing of samples between lanes. In other words, it is possible to suppress bands of samples flowing between adjacent lanes from overlapping, and quantitative analysis from being hindered, as mentioned above.

In addition, with the gel cassette 22 according to the present embodiment, at least a part of the warping prevention portion 20 is inserted between the ends in the electrophoresis direction D of the first insulating portion 13a and second insulating portion 13b. According to this configuration, since it is possible to suitably suppress deformation of the end face 19b of the gel 19, it is possible to further reduce the negative influence on discharging of sample from the end face 19b, and thus perform more accurate analysis.

In addition, with the gel cassette 22 according to the present embodiment, similarly to the gel cassette 22 of the first and second embodiments, at least a part of the warping prevention parts 20a to 20g (warping prevention parts 20c and 20d) is inserted at the central part in the width direction W that is orthogonal to the electrophoresis direction D. According to this configuration, it is possible to suitably suppress the first insulating portion 13a and second insulating portion 13b from warping by reinforcing the central parts of the first insulating portion 13a and second insulating portion 13b, which easily warp. It is thereby possible to perform more accurate analysis.

### (Fourth Embodiment)

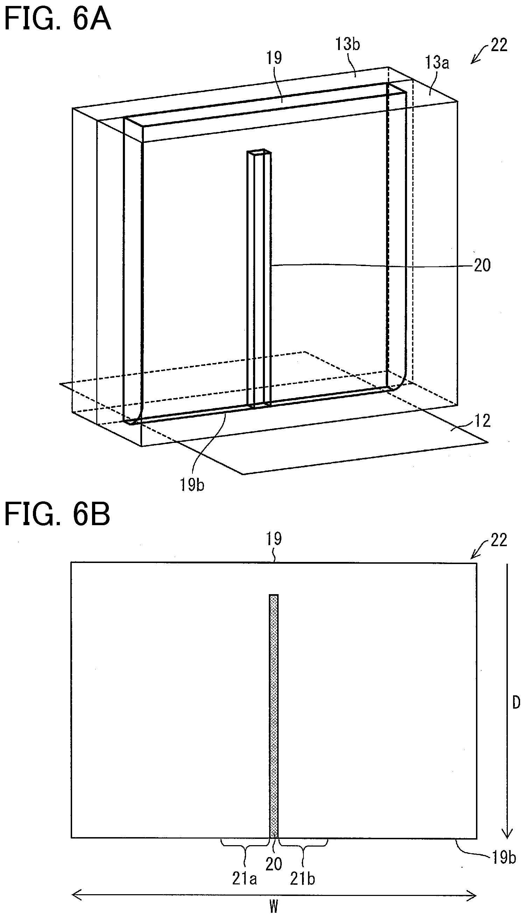

Another embodiment (fourth embodiment) of the present invention is as follows when explaining based on FIG. 6. It should be noted that, for convenience of explanation, the same reference symbols are assigned for members having the same function as members explained in the embodiment, and explanations thereof will be omitted. FIG. 6 provides views showing the shape of the gel cassette 22 according to an embodiment (fourth embodiment) of the present invention, with (a) being a perspective view showing an outline structure of the gel cassette 22 and transfer membrane 12, and (b) being a front view of the gel cassette 22.

As shown in FIGS. 6(a) and (b), the warping prevention portion 20 is inserted at the central part in the width direction W that is orthogonal to the electrophoresis direction D.

According to this configuration, by the warping prevention portion 20 being inserted at the central part in the width direction D that is orthogonal to the electrophoresis direction D, it is possible to suitably suppress the first insulating portion 13a and second insulating portion 13b from warping by reinforcing the central parts of the first insulating portion 13a and second insulating portion 13b, which easily warp. It is thereby possible to perform more accurate analysis.

In addition, with the gel cassette 22 according to the present embodiment, similarly to the gel cassette 22 of the third embodiment, at least a part of the warping prevention portion 20 is inserted between the ends in the electrophoresis direction D of the first insulating portion 13a and second insulating portion 13b. According to this configuration, since it is possible to suitably suppress deformation of the end face 19b of the gel 19, it is possible to further reduce the negative influence on discharging of sample from the end face 19b, and thus perform more accurate analysis.

### (Supplement)

A separation medium cassette for sample separation adsorption according to a first aspect of the present invention includes: a separation medium that separates sample by way of electrophoresis and discharges from an end face thereof; a first insulating portion and a second insulating portion that form a pair sandwiching the separation medium; and a warping prevention portion that is inserted between the first insulating portion and the second insulating portion, and suppresses the first insulating portion and the second insulating portion from warping so as to approach each other.

According to the above-mentioned configuration, a separation medium cassette for sample separation adsorption is provided. With the sample separation adsorption method, in order to continuously transfer the sample discharged from the end face of the separation medium to the transfer membrane, the transfer membrane is moved so as to pass through a position opposing the end face of the separation medium. At this time, for movement of this transfer membrane, force is applied to the insulating portions retaining the separation medium, and the insulating portions may warp. If the insulating portions warp, the shape of the separation medium will also change, and there is concern over influencing the separation and discharge of sample.

In contrast, according to the above-mentioned configuration, even in a case of a force such that brings together is acting on the first insulating portion and second insulating portion, the warping prevention portion inserted between the first insulating portion and second insulating portion works like a prop to suppress the first insulating portion and second insulating portion from warping. It is thereby possible to perform more accurate analysis.

According to a second aspect of the present invention, in the separation medium cassette for sample separation adsorption of the first aspect, at least a part of the warping prevention portion may be inserted between ends in a direction of the electrophoresis of the first insulating portion and the second insulating portion.

According to the above-mentioned configuration, since the warping prevention portion is inserted at the ends of the first insulating portion and second insulating portion, it is possible to more suitably suppress the end face of the separation medium from deforming. It is thereby possible to further reduce the negative influence on separation of the sample in the separation medium and discharging of sample from the end face of the separation medium, and thus perform more accurate analysis.

According to a third aspect of the present invention, in the separation medium cassette for sample separation adsorption of the first or second aspect, at least a part of the warping prevention portion may be inserted at a central part in a width direction that is orthogonal to the direction of the electrophoresis.

According to the above-mentioned configuration, by at least a part of the warping prevention portion being inserted at a central part in the width direction that is orthogonal to the electrophoresis direction, it is possible to suitably suppress the first insulating portion and second insulating portion from warping by reinforcing the central parts of the first insulating portion and second insulating portion, which easily warp. It is thereby possible to perform accurate analysis.

According to a fourth aspect of the present invention, in the separation medium cassette for sample separation adsorption of the first to third aspects, the separation medium may have a plurality of lanes, and separates the sample into each of the lanes, and the warping prevent portion may be inserted between lanes that are adjacent.

According to the above-mentioned configuration, the warping prevention portion is inserted at a position not inhibiting the separation of sample flowing in the lanes or the discharge of separated sample. It is thereby possible to perform accurate analysis.

According to a fifth aspect of the present invention, in the separation medium cassette for sample separation adsorption of the fourth aspect, the warping prevention portion may have a shape thicker at one end along the direction of the electrophoresis.

According to the above-mentioned configuration, with the lanes at both sides of the warping prevention portion having a shape thicker at one end along the electrophoresis direction, the width of the lane narrows as advancing in the electrophoresis direction. Therefore, the sample is led to a narrower space as advancing within the lane, and concentrated. The distance from the samples flowing in adjacent lanes is thereby ensured. In the above way, it is possible to concentrate the sample and prevent the mixing of samples between lanes, and thus perform more detailed analysis.

According to a sixth aspect of the present invention, in the separation medium cassette for sample separation adsorption of the fourth or fifth aspect, the warping prevention portion may be provided so as to divide between the lanes that are adjacent.

According to the above-mentioned configuration, the sample is separated and discharged without crossing between lanes. It is thereby possible to perform more detailed analysis by preventing the mixing of sample between lanes.

An analysis device for sample separation adsorption according to a seventh aspect of the present invention includes: the separation medium cassette for sample separation adsorption according to any one of the first to sixth aspects; and a moving part that causes a transfer membrane to move so as to pass through a position opposing an end face in the direction of the electrophoresis of the separation medium.

Similar effects as the above-mentioned first to sixth aspects are exerted according to the above-mentioned configuration.

The present invention is not to be limited the aforementioned embodiment, with various modifications being possible within the scope indicated by the claims, and embodiments obtained by appropriately combining the technical means disclosed in each of the different embodiments are also included in the technical scope of the present invention. Furthermore, it is possible to form novel technical features by combining the technical means disclosed in each of the respective embodiments.

### INDUSTRIAL APPLICABILITY

The present invention can be widely applied in fields that separate and analyze samples.

### EXPLANATION OF REFERENCE NUMERALS

1 analysis device (analysis device for sample separation adsorption)
10 cathode
11 anode
12 transfer membrane
13a first insulating portion
13b second insulating portion
14a buffer solution tank
14b buffer solution tank
15 arm (moving part)
16 motor (moving part)
17 power unit
18 sample introduction port
19 gel (separation medium)
19a end face
19b end face
20 warping prevention portion
21 lane
22 gel cassette (separation medium cassette for sample separation adsorption)
23 connecting part

## Claims

1. A separation medium cassette for sample separation adsorption, comprising:
a separation medium that separates sample by way of electrophoresis and discharges from an end face thereof;
a first insulating portion and a second insulating portion that form a pair sandwiching the separation medium; and
a warping prevention portion that is inserted between the first insulating portion and the second insulating portion, and suppresses the first insulating portion and the second insulating portion from warping so as to approach each other.

2. The separation medium cassette for sample separation adsorption according to claim 1, wherein at least a part of the warping prevention portion is inserted between ends in a direction of the electrophoresis of the first insulating portion and the second insulating portion.

3. The separation medium cassette for sample separation adsorption according to claim 1 or 2, wherein at least a part of the warping prevention portion is inserted at a central part in a width direction that is orthogonal to the direction of the electrophoresis.

4. The separation medium cassette for sample separation adsorption according to any one of claims 1 to 3,
wherein the separation medium has a plurality of lanes, and separates the sample into each of the lanes, and
wherein the warping prevent portion is inserted between lanes that are adjacent.

5. The separation medium cassette for sample separation adsorption according to claim 4, wherein the warping prevention portion has a shape thicker at one end along the direction of the electrophoresis.

6. The separation medium cassette for sample separation adsorption according to claim 4 or 5, wherein the warping prevention portion is provided so as to divide between the lanes that are adjacent.

7. An analysis device for sample separation adsorption comprising:
the separation medium cassette for sample separation adsorption according to any one of claims 1 to 6; and
a moving part that causes a transfer membrane to move so as to pass through a position opposing an end face in the direction of the electrophoresis of the separation medium.
